# EUROPÄISCHE PATENTSCHRIFT

(11) **EP 0 858 602 B1**
(45) Veröffentlichungstag und Bekanntmachung des Hinweises auf die Patenterteilung: **08.12.2004**
(21) Anmeldenummer: 96934176.7
(22) Anmeldetag: 30.10.1996
(51) Int. Cl.: G01N 33/00

(54) **VERFAHREN ZUR BEWERTUNG THERMISCHER UND DIELEKTRISCHER BEANSPRUCHUNG VON ELEKTRISCHEN MASCHINEN UND ANLAGEN**
PROCESS FOR EVALUATING THERMAL AND DIELECTRIC STRAIN OF ELECTRICAL MACHINES AND SYSTEMS
PROCEDE D'EVALUATION DE CONTRAINTES THERMIQUES ET DIELECTRIQUES DE MACHINES ET INSTALLATIONS ELECTRIQUES

(30) Priorität: 02.11.1995 AT 181395
(43) Veröffentlichungstag der Anmeldung: 19.08.1998
(73) Patentinhaber: VA TECH HYDRO GmbH, 1141 Wien (AT)
(72) Erfinder: MÜLLER, Franz, A-8071 Grambach (AT); MUSSBACHER, Günther, A-8062 Kumberg (AT)
(74) Vertreter: VA TECH Patente GmbH & Co
(86) Internationale Anmeldenummer: PCT/AT1996/000208
(87) Internationale Veröffentlichungsnummer: WO 1997/016725

(56) Entgegenhaltungen:
- WO-A-93/25901

## Beschreibung

Die Erfindung betrifft ein Verfahren zur Bewertung thermischer und dielektrischer Beanspruchung von elektrischen Maschinen und Anlagen, insbesondere rotierende Maschinen, welche Komponenten aus Isolierstoffen und mit Überzugslacken versehene Maschinenteile aufweisen, wobei die Maschinen mit einem Kühlkreislauf ausgerüstet sind und mittels einem gasförmigen Kühlmedium gekühlt werden, und das gasförmige Kühlmedium kontinuierlich oder nach wählbaren Zeitintervallen analysiert wird, und zwar derart, daß gasförmige Spaltprodukte und Zersetzungsprodukte, hervorgerufen durch eine thermische Beanspruchung der Isolierstoffe bzw. Überzugslacke, sowie Ozon, hervorgerufen durch eine dielekrische Beanspruchung, bestimmt und gemessen werden, und diese Analysenwerte gemeinsam bewertet werden, und dadurch der momentane Betriebszustand der Maschine festgestellt wird.

Ein derartiges Verfahren ist aus der EP-A 0 646 240 bekannt.

Die immer höher werdende Maschinenausnutzung, sowie die steigenden Anforderungen an die Betriebssicherheit rotierender elektrischer Maschinen erfordern immer bessere Überwachungsmethoden für die aktiven Teile der Maschine, welche das Wicklungssystem, einschließlich Schaltverbindungen und Schaltleitungen, sowie das Blechpaket sind.
Bei rechtzeitiger Feststellung von Veränderungen an wesentlichen Maschinenkomponenten, hauptsächlich der Isolation, an diesen Teilen, können geeignete Gegenmaßnahmen ergriffen werden, und somit größere Schäden und längere Betriebsausfälle vermieden werden.

Derzeit erfolgt eine Überwachung elektrischer Maschinen, sieht man von der Schwingungsüberwachung ab, fast ausschließlich nur hinsichtlich des thermischen Zustandes des Wicklungssystemes im Ständer der Maschine.

Bei Maschinen mit Hochspannungswicklungen wirkt sich vor allem der Umstand nachteilig aus, daß die Temperatursensoren nur dort angebracht werden können, wo keine dielektrische Beanspruchung durch die Hochspannung gegeben ist. Aus diesem Grunde werden daher die Sensoren nur im Nutbereich der Wicklung, wo durch den Außenglimmschutz keine dielektrische Beanspruchung auftritt, angeordnet.
Diese Art der Temperaturüberwachung hat den Nachteil, daß nur punktuell und kleine Abschnitte vom gesamten Wicklungssystem überwacht werden kann.

Die in den letzten Jahren entwickelte Meßtechnik mit einer Datenübertragung über Lichtwellenleiter bietet zwar den Vorteil der Einsetzbarkeit unter dielektrischer Beanspruchung, hat jedoch den Nachteil, daß sie auch nur eine punktuelle Überwachung darstellt. Weiters sind oft die Kosten für solche Systeme wirtschaftlich nicht vertretbar.

In der US-PS 3 427 880 ist ein Verfahren und eine Anordnung zur Ermittlung der Überhitzung von Teilen in gasgekühlten elektrischen Maschinen beschrieben, wobei die Maschinenteile, insbesondere Blechlamellen, mit einem Überzugsmaterial versehen sein müssen, welche bei Überhitzung kleinste Partikelchen freisetzen, die dann mit geeigneten Detektoren gemessen werden. Der Detektor ist hier eine Ionisationskammer. Als Überzugsmaterial werden Polymere eingesetzt, wobei bei Erreichen einer bestimmten Temperatur durch Zerfall kleinste Partikelchen freigesetzt werden. Darüber hinaus werden als Indikator oder als Zusatzstoffe radioaktive Komponenten eingesetzt. Nachteilig dabei ist, daß die Überwachung nicht On-Line erfolgt.

Die Aufgabe der Erfindung besteht nun darin, ein zuverlässiges permanentes Verfahren zur globalen Überwachung der aktiven Teile der elektrischen Maschine, insbesondere Bechpaket, Wicklungssystem, elektrische Schalt- und Schraubverbindungen, Lötstellen, usw. zu schaffen.

Die Aufgabe wird durch die Erfindung gelöst. Diese ist dadurch gekennzeichnet, daß zusätzlich eine Feuchtigkeitsmessung des gasförmigen Kühlmediums erfolgt, und daß ein empirisch oder rechnerisch ermitteltes Referenzspektrum von Feuchte (Temperatur|mg H₂O/kg), Dieletrikum (kV|µg O₃/kg) und der Zusammensetzung der thermischen Zersetzungsprodukte (Temperatur|mg CH/kg) abgespeichert ist, und zyklisch ein Vergleich zwischen den aktuellen erfaßten Meßwerten und dem Referenzspektrum erfolgt, wobei die Auswertung der Messung On-Line durchgeführt wird. Dadurch wird der Feuchtegehalt des Kühlmediums bei der Messung mitberücksichtigt. Damit kann auf eine etwaige Betauung von hochspannungsführenden Maschinenkomponenten rückgeschlossen werden. Dies ist insbesondere beim Betrieb von Maschinen in äquatorialen Ländern von Vorteil. Weiters können mit dieser Methode Kühlerleckagen frühzeitig erfaßt werden. Es erfolgt immer ein Vergleich der Zersetzungsprodukte im Kühlmedium im Urzustand der Maschine mit den aktuellen Daten, wodurch immer der momentane Zustand der Isolierung der aktiven Teile der Maschine vorliegt. Bei einer Detektion eines Fehlers kann die Maschine durch diese Methode der Überwachung des Kühlmediums auch immer im unkritischen Leistungsbereich weiter betrieben werden.

Von Vorteil ist, daß über die Länge der Kette der Zersetzungsprodukte im Kühlmedium, insbesondere der Kohlenwasserstoffe, der thermische Zustand bzw. die Alterung der Isolierstoffe bestimmt wird. Bei einer neuen Maschine sind die Kohlenwasserstoffketten eher kurz, z.B. CH₄. Mit zunehmender Betriebsdauer werden diese Ketten immer länger, z.B. C₄H₁₀. Es kann somit vorausgesagt werden wann eine Revision der Maschine erforderlich ist

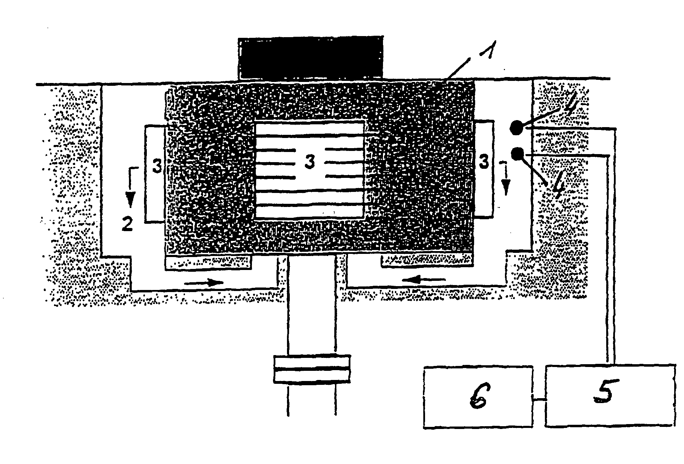

Die Erfindung wird anhand der Zeichnung noch näher erläutert.

Die Fig. zeigt schematisch eine rotierende elektrische Maschine mit einem geschlossenen Kühlkreislauf. Das von der Maschine 1 erwärmte Kühlmedium 2 wird in den Kühlem 3 abgekühlt und in den Kreislauf wieder zurückgeführt. In diesem Kühlkreislauf sind an geeigneter Stelle, vorzugsweise nach den Kühlern 3 die Sensoren 4 zur Analyse des Kühlmediums 2 angebracht. Im Auswertegerät 5 werden die Daten analysiert, bewertet, eventuelle Trends ermittelt und an die Anzeige 6 übergeben.

Jede signifikante Änderung der gasförmigen Zusammensetzung des Kühlmediums 2 bzw. das stärkere Auftreten einzelner spezifischer Gaskomponenten ist nach einem Vergleich mit einem Referenzspektrum ein Hinweis auf eine Überlastung oder sich anbahnender Defekt eines Maschinenbauteiles.

## Patentansprüche

1. Verfahren zur Bewertung thermischer und dielektrischer Beanspruchung von elektrischen Maschinen und Anlagen, insbesondere rotierende Maschinen, welche Komponenten aus Isolierstoffen und mit Überzugslacken versehene Maschinenteile aufweisen, wobei die Maschinen mit einem Kühlkreislauf ausgerüstet sind und mittels einem gasförmigen Kühlmedium gekühlt werden, und das gasförmige Kühlmedium kontinuierlich oder nach wählbaren Zeitintervallen analysiert wird, und zwar derart, daß gasförmige Spaltprodukte und Zersetzungsprodukte, hervorgerufen durch eine thermische Beanspruchung der Isolierstoffe bzw. Überzugslacke, sowie Ozon, hervorgerufen durch eine dielektrische Beanspruchung, bestimmt und gemessen werden, und diese Analysenwerte gemeinsam bewertet werden, und dadurch der momentane Betriebszustand der Maschine festgestellt wird, **dadurch gekennzeichnet, daß** zusätzlich eine Feuchtigkeitsmessung des gasförmigen Kühlmediums erfolgt, und daß ein empirisch oder rechnerisch ermitteltes Referenzspektrum von Feuchte (Temperatur|mg H₂O/kg), Dielektrikum (kV|µg O₃/kg) und der Zusammensetzung der thermischen Zersetzungsprodukte (Temperatur|mg CH/kg) abgespeichert ist, und zyklisch ein Vergleich zwischen den aktuellen erfaßten Meßwerten und dem Referenzspektrum erfolgt, wobei die Auswertung der Messung On-Line durchgeführt wird.

2. Verfahren nach Anspruch 1, **dadurch gekennzeichnet, daß** über die Länge der Kette der Zersetzungsprodukte, welche Kohlenwasserstoffe sind, im Kühlmedium, der thermische Zustand bzw. die Alterung der Isolierstoffe bestimmt wird.

## Claims

1. A process for evaluating the thermal and dielectric stressing of electrical machines and installations, in particular, of rotating machines, containing component parts made of insulating materials and machine elements with a lacquer coating, wherein the machines are provided with a cooling circuit and are cooled by means of a gaseous cooling medium and the gaseous cooling medium is analyzed continuously or at selected intervals of time, in order to determine and measure gaseous scission and degradation products formed as a result of thermal stressing of the insulating materials and of the lacquers, as well as the ozone formed as a results of the dielectric stressing and the results of the measurements are evaluated collectively for providing an instant assessment of the operational status of the machine, **characterized in that** a moisture determination is carried out additionally in the gaseous cooling medium and **in that** an empirically or arithmetical determined reference spectra of the moisture (temperature | H₂O /kg ), dielectric (kV | µg O₃ / kg) and of the composition of the thermal degradation products (temperature | mg CH / kg) is stored and a comparison is carried out at intervals between the current measured values and the reference spectra, whereat the evaluation of the measurements is carried out on-line.

2. A process according to claim 1, **characterized in that** the thermal state or the ageing of the insulating materials is determined from the chain length of the degradation products, which are hydrocarbons, in the cooling medium.

## Revendications

1. Procédé pour évaluer l'état de contrainte thermique de machines et d'installations électriques et l'état de contrainte de leurs diélectriques et, en particulier, de machines rotatives, présentant des composants réalisés en matériaux isolants et des composants de machine avec un revêtement en laque, où les machines sont pourvues d'un circuit de refroidissement et sont refroidies par un milieu de refroidissement gazeux et le milieu de refroidissement gazeux est analysé de manière continue ou à des intervalles de temps choisis, dans le but de déterminer et de mesurer les produits de scission et de dégradation formés par suite des contraintes thermiques s'exerçant sur les matériaux isolants et les laques, ainsi que l'ozone formé par suite des contraintes s'exerçant sur les diélectriques et les résultats des mesures sont évalués collectivement pour fournir une évaluation instantanée de l'état de fonctionnement de la machine, **caractérisé en ce qu**'une détermination de l'humidité est effectuée en plus dans le milieu de refroidissement gazeux et **en ce que** des spectres de référence, empiriques ou calculés de l'humidité (température | H₂O /kg ), du comportement des diélectriques (kV | µg O₃ / kg) et de la composition des produits de dégradation thermique (température | mg CH / kg) sont conservés et qu'une comparaison est effectuée à des intervalles de temps entre les valeurs couramment mesurées et les spectres de référence, ce qui permet un traitement des mesures en direct.

2. Procédé selon la revendication 1, **caractérisé en ce que** l'état thermique ou l'altération des matériaux isolants est déterminé à partir de la longueur de chaîne des produits de dégradation, qui sont des hydrocarbures, dans le milieu de refroidissement.
